# EUROPEAN PATENT SPECIFICATION

(11) **EP 3 254 572 B1**
(45) Date of publication and mention of the grant of the patent: **04.11.2020**
(21) Application number: 16746052.6
(22) Date of filing: 15.01.2016
(51) Int. Cl.: A24F 47/00, B65D 47/26

(54) **ATOMIZER, ELECTRONIC CIGARETTE AND LIQUID STORAGE DEVICE SUITABLE FOR BEING REPLACED**
ZERSTÄUBER, ELEKTRONISCHE ZIGARETTE UND ERSETZBARE FLÜSSIGKEITSSPEICHERVORRICHTUNG
ATOMISEUR, CIGARETTE ÉLECTRONIQUE ET DISPOSITIF DE STOCKAGE DE LIQUIDE POUVANT ÊTRE REMPLACÉ

(30) Priority: 04.02.2015 CN 201510057585
(43) Date of publication of application: 13.12.2017
(73) Proprietor: Philip Morris Products S.A., 2000 Neuchâtel (CH)
(72) Inventor: LI, Yonghai, Shenzhen Guangdong 518103 (CN); XU, Zhongli, Shenzhen Guangdong 518103 (CN); DUAN, Hongxing, Shenzhen Guangdong 518103 (CN); ZHANG, Yansheng, Shenzhen Guangdong 518103 (CN)
(74) Representative: Gritschneder, Sebastian
(86) International application number: PCT/CN2016/000025
(87) International publication number: WO 2016/124057

(56) References cited:
- EP-A1- 3 047 743
- CN-A- 104 082 863
- CN-A- 104 082 863
- CN-A- 104 305 526
- CN-A- 104 738 816
- CN-U- 203 353 681
- CN-U- 204 070 581
- CN-U- 204 120 222
- CN-U- 204 444 256
- CN-U- 204 444 256
- CN-U- 204 907 926
- US-A1- 2014 261 495

## Description

### Technical field

The present invention relates to the field of novel smoking equipment, in particular to a replaceable liquid storage device for use in an electronic cigarette.

### Background art

The main function of an atomizer, as an important constituent part of an electronic cigarette, is to atomize the internal e-liquid to form an aerosol for inhaling, and thereby simulate the smoke mist generated by a traditional cigarette; the harm to the human body can be reduced significantly compared with the traditional method of burning tobacco. At present, most atomizers comprise a liquid storage device for storing e-liquid and an atomizing assembly connected to the liquid storage device. Atomizers of this type on the market are divided into two main categories; in one category, the liquid storage device and the atomizing assembly form a one-piece structure, while in the other category, the liquid storage device and the atomizing assembly form a two-piece structure. However, atomizers with these two categories of structure both exhibit the following deficiencies during use.

In the prior art, atomizers with one-piece structures are sub-divided into disposable and multiple-use atomizers. Since the e-liquid capacity inside a disposable atomizer is limited, the entire atomizer (including the liquid storage device and the atomizing assembly) will be discarded when the e-liquid is used up. As a result, disposable atomizers are very expensive to use, and cause environmental pollution. When the e-liquid in a multiple-use atomizer is used up, it is necessary to use a special e-liquid bottle to refill the atomizer with e-liquid so operation is bothersome, which causes inconvenience to the user.

To solve the above problem, an atomizer with a two-piece structure has appeared in the prior art. The liquid storage device and the atomizing assembly in this atomizer are detachably connected, and when the e-liquid is used up, all that need be done is to replace the liquid storage device; the atomizing assembly can be used repeatedly. At present, a relatively typical liquid storage device in an atomizer with a two-piece structure employs tin foil to seal the e-liquid; a piercing element is provided on the atomizing assembly, and when the two are connected, the tin foil can be pierced using the piercing element so that e-liquid flows into the atomizing assembly. However, the main deficiency of this solution is that: tin foil that has been pierced cannot be restored to its original state, and when the liquid storage device needs to be replaced, the residual e-liquid in the liquid storage device will spill out copiously when the user detaches the liquid storage device from the atomizing assembly, contaminating the exterior of the atomizer while giving the consumer a poor user experience.

CN 104 082 863 A is directed to an electronic cigarette comprising a mouthpiece, a connection element, a liquid storage tube and a heat generating component that are connected in sequence. The liquid storage tube comprises a partition plate that divides the liquid storage into a lower cavity and an upper cavity. By rotating the partition plate the amount of tobacco liquid that is provided from the lower cavity to the heat generating component can be adjusted. When the lower cavity is depleted, new tobacco liquid can be provided from the upper cavity by rotating the partition plate. New tobacco liquid can be injected into the liquid storage tube via holes in a liquid injection plate, without any need to detach the liquid storage tube.

CN 104 305 526 A is directed to an electronic cigarette comprising a cartridge body including a e-liquid reservoir. The cartridge body is at least partially made from transparent material.

### Content of the invention

The technical problem to be solved by the present invention is to overcome the deficiencies of the prior art by providing a low-cost atomizer which can prevent spillage of e-liquid when the liquid storage device is detached.

To solve the above technical problem, the technical solution employed in the present invention is as defined in independent claim 1. An atomizer comprises a liquid storage device according to the present invention which is used for storing e-liquid and has an open end, and an atomizing assembly which is detachably connected to the open end, the atomizing assembly comprising an atomizing chamber and an atomizing unit disposed in the atomizing chamber, the atomizing unit being used to atomize e-liquid to form an aerosol for inhaling; the atomizing assembly has a connection part for connecting to the liquid storage device, a liquid inlet in communication with the atomizing chamber is provided on the connection part, a seal having a liquid outlet is provided on the open end, and the connection part is suitable for inserting into the open end of the liquid storage device and then rotating by a predetermined angle so as to engage and connect with the liquid storage device; a rotary component capable of rotating together with the connection part is also provided on the open end, the rotary component abutting the seal; when rotating from an initial insertion position to an engaged position, the connection part can drive the rotary component to rotate so as to connect the liquid outlet to the liquid inlet; when rotating back to the initial position, the connection part can drive the rotary component to rotate so that the rotary component blocks the liquid outlet.

As a specific form of the rotational engagement and connection, at least one projecting part is provided on an end face of the connection part, the liquid inlet being provided on the projecting part, an accommodating hole adapted in shape to the projecting part is provided on the rotary component, and when the connection part is inserted into the open end of the liquid storage device, the projecting part can insert into the accommodating hole and abut the seal.

Furthermore, a connection ring is fixedly fitted onto the open end of the liquid storage device, the rotary component is received in the connection ring so as to be capable of relative rotation, at least one protruding engager is provided on a sidewall of the connection part, and on an inner wall of the connection ring are provided a guide slot to receive the protruding engager, and an inwardly extending step, the step being configured to engage and connect with the protruding engager.

Preferably, a projecting locating part is provided on the outside of the rotary component, and locating slots matching the locating part are provided on the inside of the connection ring to correspond to the initial insertion position and the engaged position of the connection part, respectively.

Further preferably, the locating slots which correspond to the initial position and the engaged position form an arc angle of 90 degrees on an inside wall of the connection ring.

Furthermore, an annular sealing cushion is provided on an edge of the liquid outlet, on that end face of the seal which abuts the rotary component.

Furthermore, a mouthpiece which is integrally formed with the housing of the liquid storage device is provided on the liquid storage device, a gas guide tube in communication with the mouthpiece is also provided inside the liquid storage device, and an aerosol generated in the atomizing chamber can pass through the gas guide tube and be sucked out through the mouthpiece.

Furthermore, a gas flow outlet in communication with the atomizing chamber is provided in the centre of the connection part, while an axial tube part extending axially is provided in the centre of the seal, the gas flow outlet being in communication with the gas guide tube via the axial tube part.

As a particular embodiment, the atomizing unit comprises a liquid guide body and a heating element in contact with the liquid guide body, an end of the liquid guide body being used to absorb e-liquid flowing in through the liquid inlet, and the heating element heating the e-liquid so that the latter evaporates to form an aerosol.

Furthermore, a layer of a fibrous material forming a liquid storage body is also provided between an end of the liquid guide body and the liquid inlet.

The present disclosure also provides an electronic cigarette, comprising an atomizer and a power supply assembly; the atomizer may be the atomizer according to the various particular solutions and preferred improved solutions; the power supply assembly is connected to the atomizing assembly and provides a power source for the internal atomizing unit.

The present invention also provides a replaceable liquid storage device that is connected to an atomizing assembly, the interior of the liquid storage device being used to store e-liquid; the liquid storage device has an open end on which are provided a seal having a liquid outlet and a rotary component which abuts the seal; when the liquid storage device is not connected to the atomizing assembly, the rotary component is used to block the liquid outlet so as to seal e-liquid inside; when the liquid storage device is connected to the atomizing assembly, the rotary component can rotate and open the liquid outlet.

Preferably, the housing of the liquid storage device is made of a transparent material.

The beneficial effects of the present invention are as follows: Since the atomizing assembly and the liquid storage device in the atomizer according to the present invention are detachably connected, when the e-liquid has been used up, all that need be done is to discard the lower-cost liquid storage device and replace it with a new one, while the higher-cost atomizing assembly can be used repeatedly, enabling a huge reduction in the cost of use. In addition, the atomizing assembly has a connection part for connecting to the liquid storage device, the connection part is suitable for inserting into the open end of the liquid storage device and then rotating by a predetermined angle so as to engage and connect with the liquid storage device, and a seal and a rotary component are provided on the open end. In a normal state, when the atomizing assembly is not connected to the liquid storage device, the rotary component can block the liquid outlet on the seal, to ensure that no e-liquid leaks from the liquid storage device during warehouse storage or transportation. When engaging and connecting with the liquid storage device, the connection part of the atomizing assembly can drive the rotary component to rotate so as to open the liquid outlet, so that e-liquid flows smoothly into the atomizing assembly. When disengaging from the liquid storage device, the connection part can drive the rotary component to rotate to the initial position in which the liquid outlet is sealed, so that the e-liquid inside is sealed again, ensuring that no e-liquid will spill from the liquid storage device during detachment, to give the consumer a good user experience.

### Description of the accompanying drawings

Fig. 1 is a schematic diagram of the structure of the liquid storage device in an embodiment;
Fig. 2 is a schematic diagram of the structure of the atomizing assembly in an embodiment;
Fig. 3 is a schematic exploded view of the structure of the liquid storage device in an embodiment;
Fig. 4 is a schematic diagram of the structure of the connection ring on the liquid storage device in an embodiment;
Fig. 5 is an internal sectional view of the atomizing assembly in an embodiment;
Fig. 6 is a schematic exploded view of the structure of the atomizing assembly in an embodiment;
Fig. 7 is a schematic diagram of the state in which the rotary component is blocking the liquid outlet on the seal in an embodiment;
Fig. 8 is a schematic diagram of the state in which the atomizing assembly has driven the rotary component to rotate and opened the liquid outlet on the seal in an embodiment;
Fig. 9 is a schematic diagram of the structure of the electronic cigarette in an embodiment.

### Particular embodiments

The atomizer disclosed in the present disclosure is mainly suitable for electronic cigarettes, liquid drug ingredient volatilizing devices or other aromatic ingredient releasing devices. In this embodiment, the example of an electronic cigarette will be described. The atomizer consists of a liquid storage device and an atomizing assembly which are detachably connected together; a rotational engagement and connection structure is used therebetween to enable opening or closing of a liquid flow channel therebetween, achieving the objective of preventing leakage of e-liquid to the outside when the liquid storage device is detached. In addition, the electronic cigarette provided by the present disclosure consists of three parts, namely a power supply assembly, an atomizing assembly and a liquid storage device. Since the e-liquid in the liquid storage device is a consumable, the liquid storage device must be replaced frequently during use by the consumer so its service life is relatively short, the atomizing assembly being second in service life, and the power supply assembly having the longest service life, for it is not easily damaged and does not age easily. The atomizing assembly can be used repeatedly, effectively reducing the cost of use. It can of course be appreciated that if the atomizing assembly is damaged, it can also be replaced with a new atomizing assembly matching the power supply assembly. The structure and principles of use of the atomizer described above, the electronic cigarette in which the atomizer is used, and the replaceable liquid storage device are expounded further below by way of particular embodiments.

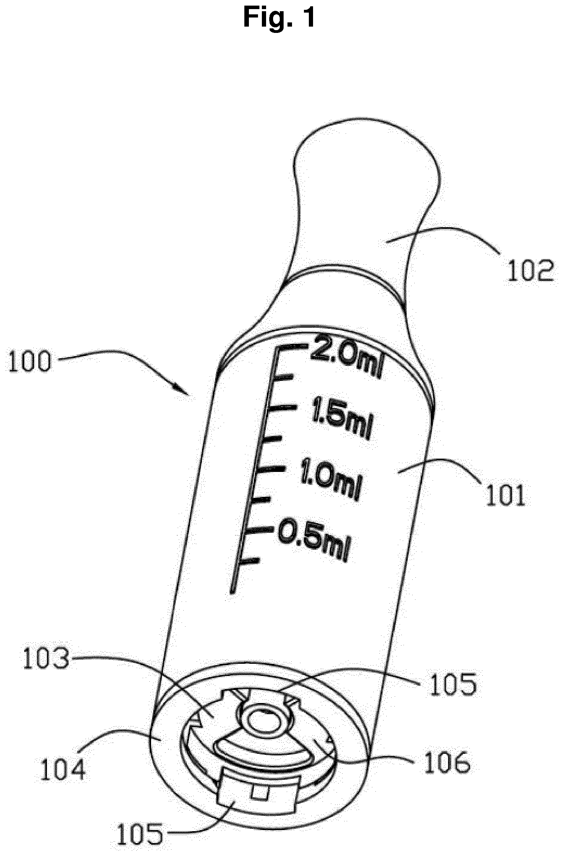

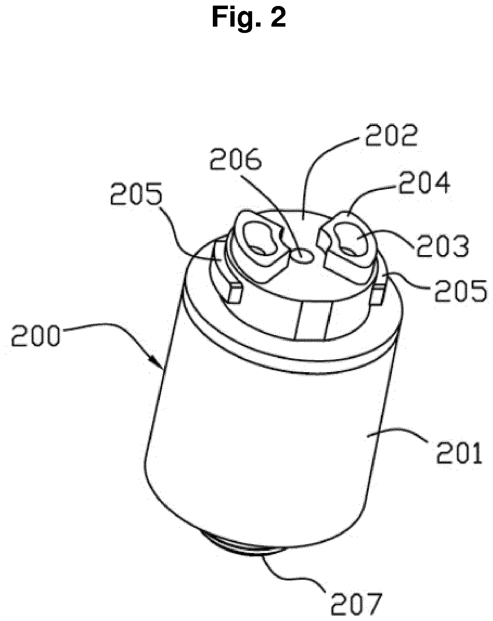

Referring to Figs. 1 and 2, this embodiment provides an atomizer for an electronic cigarette, the atomizer mainly comprising two parts, a liquid storage device 100 and an atomizing assembly 200 which are detachably connected together; Fig. 1 and Fig. 2 show the external structures of the liquid storage device 100 and atomizing assembly 200, respectively. The liquid storage device 100 comprises a housing 101 and a mouthpiece 102 located at one end of the housing 101; the mouthpiece 102 and housing 101 are integrally formed into a unit, and a chamber for storing e-liquid is provided inside the housing 101. In a preferred solution of this embodiment, the housing 101 is made of a transparent material, e.g. glass or transparent plastic; the user can observe how much e-liquid remains inside through the housing 101, and a scale for showing the amount of e-liquid remaining is also provided on the housing 101. The other end of the housing 101 is an open end 103; the atomizing assembly 200 is detachably connected to the open end 103, and a rotary component 106 is provided inside the open end 103. In a normal state, the rotary component 106 is used to seal the e-liquid inside the liquid storage device 100; only when the atomizing assembly 200 is connected to the open end 103 will the rotary component 106 be driven to rotate, such that the e-liquid inside the liquid storage device 100 flows smoothly into the atomizing assembly 200.

The atomizing assembly 200 comprises an outer sleeve 201; inside the outer sleeve 201 are provided an atomizing chamber and an atomizing unit disposed in the atomizing chamber. The atomizing unit is used for atomizing e-liquid to form an aerosol for inhaling, and the specific structure of the atomizing unit is described below. A connection part 202 for connecting to the liquid storage device 100 is provided on the atomizing assembly 200, and a liquid inlet 203 in communication with the atomizing chamber is provided on the connection part 202; in this embodiment, there are two liquid inlets 203, arranged symmetrically with respect to each other. The connection part 202 is suitable for inserting into the open end 103 of the liquid storage device 100 and then rotating by a predetermined angle to engage and connect with the liquid storage device 100, and during rotation it can drive the rotary component 106 to rotate, releasing the e-liquid inside the liquid storage device 200, so that e-liquid enters the liquid inlets 203. The specific structure of rotational engagement and connection formed between the liquid storage device 100 and the connection part 202 is described below.

A threaded sleeve 207 is provided on the other end of the atomizing assembly 200 opposite the connection part 202; the atomizing assembly 200 can be connected to the power supply assembly by means of the threaded sleeve 207 so as to assemble the electronic cigarette. Since the atomizing assembly 200 and power supply assembly can both be used for a relatively long time, when the e-liquid in the liquid storage device 100 is used up, all that need be done is to replace the liquid storage device 100; there is no need to discard the entire atomizer. This greatly reduces the cost of use for the consumer, while also reducing the environmental pollution caused by discarding an entire atomizer.

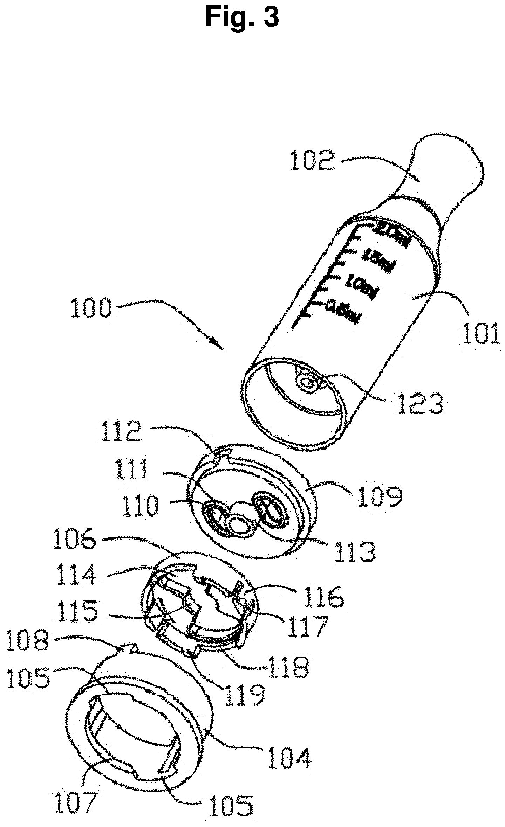

Now referring to Figs. 2 and 3, a connecting ring 104 is fixed to the open end 103 of the liquid storage device 100. The cross section of the connection ring 104 is substantially circular, and the rotary component 106 is received in the connecting ring 104 so as to be capable of relative rotation. At least one protruding engager 205 is provided on a sidewall of the connection part 202. In this embodiment, there are specifically two protruding engagers 205, arranged symmetrically with respect to a centre axis of the atomizing assembly 200. On an inner wall of the connection ring 104 are provided guide slots 105 to receive the protruding engagers 205, and inwardly extending steps 107, the steps 107 being configured to engage and connect with the protruding engagers 205. The guide slots 105 are provided in an axial direction of the liquid storage device 100, are also two in number, and are provided in positions corresponding to the protruding engagers 205; the steps 107 are arc-shaped projections disposed at an inner edge of the connection ring 104.

A seal 109 having a liquid outlet 110 is provided on the open end 103; the seal 109 is used to seal the e-liquid inside the liquid storage device 100, e-liquid only being able to flow out from inside the liquid outlet 110. Corresponding to the liquid inlets 203, there are also two liquid outlets 110 in this embodiment; it can of course be appreciated that there may also be one or more than two liquid outlets 110. The top end face of the rotary component 106 which can rotate together with the connection part 202 abuts the seal 109, for the purpose of closing the liquid outlets 110, and will only open the liquid outlets 110 when rotated to a specific position.

The process of assembling the various components of the liquid storage device 100 will be presented below. During assembly, first of all the rotary component 106 is positioned inside the connection ring 104; notches 118 of the same width as the guide slots 105 are provided on a sidewall of the rotary component 106, and during positioning, the notches 118 must be aligned with the guide slots 105, so that the protruding engagers 205 can pass through the guide slots 105 and insert into the notches 118, to enable joint motion of the rotary component 106 and the connection part 202. Then the seal 109 is fitted tightly against the rotary component 106 and installed fixedly onto the connection ring 104. Specifically, a locating post 108 is provided on a sidewall on the connection ring 104, a matching locating slot 112 is provided on a sidewall of the seal 109, and connection of the connection ring 104 to the seal 109 is accomplished by inserting the locating post 108 into the locating slot 112. Finally, the connection ring 104 is inserted into the open end 103 of the housing 101 and they are fitted in a fixed manner, so that the sealing action of the seal 109 on the open end 103 is realized. In this embodiment, the connection ring 104 is fitted to the open end 103 of the housing 101 by an interference fit.

In a preferred solution of this embodiment, at least one projecting part 204 is provided on an end face of the connection part 202. There are preferably two projecting parts 204 in this embodiment. The liquid inlets 203 are provided on these projecting parts 204, accommodating holes 114 adapted in shape to the projecting parts 204 are provided on the rotary component 106, and when the connection part 202 is inserted into the open end 103 of the liquid storage device 100, the projecting parts 204 can insert into the accommodating holes 114 and abut the seal 109; the projecting parts 204 inserting into the accommodating holes 114 can also drive the rotary component 106 to rotate. It is easy to understand that during rotation of the rotary component 106, the accommodating holes 114 and the liquid inlets 203 on the projecting parts 204 always correspond to each other in the same position. When the connection part 202 is located in an initial position just after insertion into the open end 103, the liquid outlets 110 on the seal 109 are staggered with respect to the liquid inlets 203 and the accommodating holes 114; at this time, other solid parts of the rotary component 106 seal the liquid outlets 110. Only when the connection part 202 drives the rotary component 106 to rotate to an engaged position will the liquid outlets 110 be positionally aligned and connected with the liquid inlets 203.

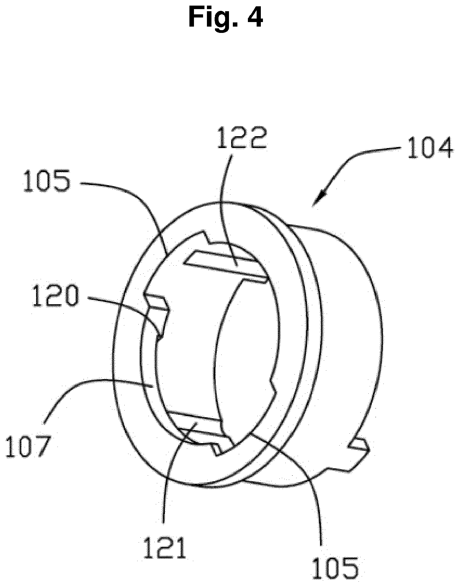

Referring to Fig. 4 in conjunction with Fig. 3, a projecting locating part 117 is provided on the outside of the rotary component 106; the locating part 117 is a small lug or projecting post which projects from an outer wall. Locating slots 122 and 121 matching the locating part 117 are provided on the inside of the connection ring 104 to correspond to the initial insertion position and the engaged position of the connection part 202, respectively. During rotation, the locating part 117 will switch between the locating slot 122 and locating slot 121, and it will be clearly felt by the fingers when the locating part rotates to the locating slot 122 or locating slot 121. Preferably, the locating part 117 is located on an elastic arm 116, with relatively small notches between the elastic arm 116 and the rotary component 106, so that when the locating part 117 disengages from the locating slot 122 or locating slot 121, the elastic arm 116 can deform somewhat. In a preferred solution of this embodiment, the locating slot 122 and locating slot 121 which correspond to the initial position and the engaged position form an arc angle of 90 degrees on an inside wall, i.e. rotation of the connection part 202 from the initial position to the engaged position requires 90 degrees of rotation.

There follows a description relating to the initial position and engaged position of the connection part 202 during rotation. Referring to Fig. 4, a limit position 120 which is slightly higher in an axial direction than the step 107 is provided on the step 107 of the connection ring 104; a limit position 119 is also provided correspondingly on an outer wall of the rotary component 106. When the protruding engager 205 on the connection part 202 is inserted into the guide slot 105 and has not yet been rotated, this is the initial position. When the connection part 202 has driven the rotary component 106 to rotate therewith by 90 degrees, the limit position 119 reaches the limit position 120 and there is mutual restriction, i.e. the limit position 120 can prevent over-rotation of the connection part 202. At the same time, the locating part 117 falls into the locating slot 121, at which time the connection part 202 reaches the engaged position; in this state, the liquid outlets 110 are connected with the liquid inlets 203. When the connection part 202 rotates in the opposite direction and returns to the initial position, the rotary component 106 rotates by 90 degrees again so as to block the liquid outlets 220. It must be explained that it is possible that the limit position 120 is not provided on the step 107 or that the connection part 202 passes the limit position during rotation, i.e. when the connection part 202 needs to be unscrewed, it may not need to be rotated reversely, rather it may be rotated by a further 90 degrees in the screw-in direction to reach the initial position so as to be detached from the liquid storage device.

Now referring to Fig. 3, in order to further enhance the sealing effect during rotation, an annular sealing cushion 111 is provided on an edge of the liquid outlet 110, on that end face of the seal 109 which abuts the rotary component 106. In this embodiment, the entire seal 109 is preferably made of a silicone rubber material; the sealing cushion 111 and the seal 109 form a one-piece structure, and the sealing cushion 111 projects from that end face of the seal 109 which abuts the rotary component 106. When the rotary component 106 blocks the liquid outlet 110, the sealing cushion 111 abuts an end face of the rotary component 106 elastically. When the liquid outlet 110 is aligned with the liquid inlet 203, the sealing cushion 111 abuts an end face of the projecting part 204 elastically, to ensure that no leakage of liquid occurs in any state.

There follows a description of a gas flow channel for discharging the aerosol from inside the atomizing assembly 200. Referring to Fig. 3, a mouthpiece 102 which is integrally formed with the housing 101 of the liquid storage device 100 is provided on the liquid storage device 100. A gas guide tube 123 in communication with the mouthpiece 102 is also provided inside the liquid storage device 100; the gas guide tube 123 also forms a one-piece structure with the housing 101, and an aerosol generated in the atomizing chamber 209 can pass through the gas guide tube 123 and be sucked out through the mouthpiece 102. It can of course be appreciated that the gas guide channel may also be formed outside the liquid storage device 100.

Preferably, a gas flow outlet 206 in communication with the atomizing chamber 209 is provided in the centre of the connection part 202, while an axial tube part 113 extending axially is provided in the centre of the seal 109, the axial tube part 113 forming a one-piece structure with the seal 109, and the gas flow outlet 206 being in communication with the gas guide tube 123 via the axial tube part 113. A rotation shaft hole 115 is provided in the centre of the rotary component 106; the rotary component 106 is fitted around the axial tube part 113 by means of the rotation shaft hole 115, and the rotation shaft hole 115 is in communication with the accommodating holes 114 on two sides. The two liquid outlets 110 on the seal 109 are arranged symmetrically with respect to the centre axis of the axial tube part 113. Driven by the connection part 202, the rotary component 106 can rotate around the centre axis of the axial tube part 113.

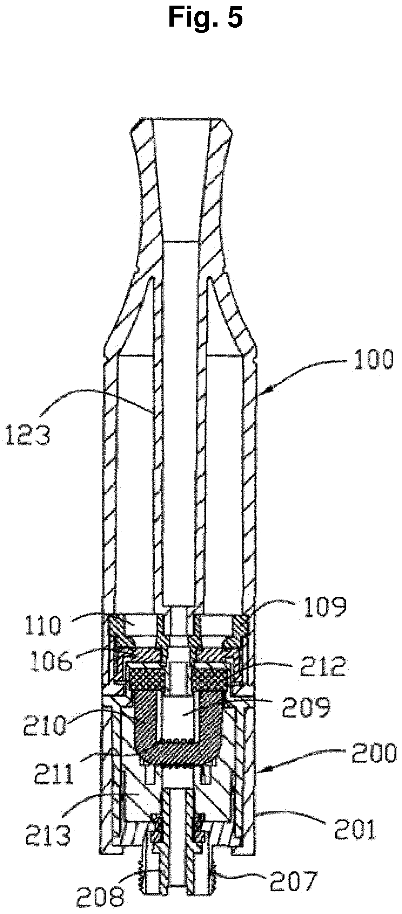

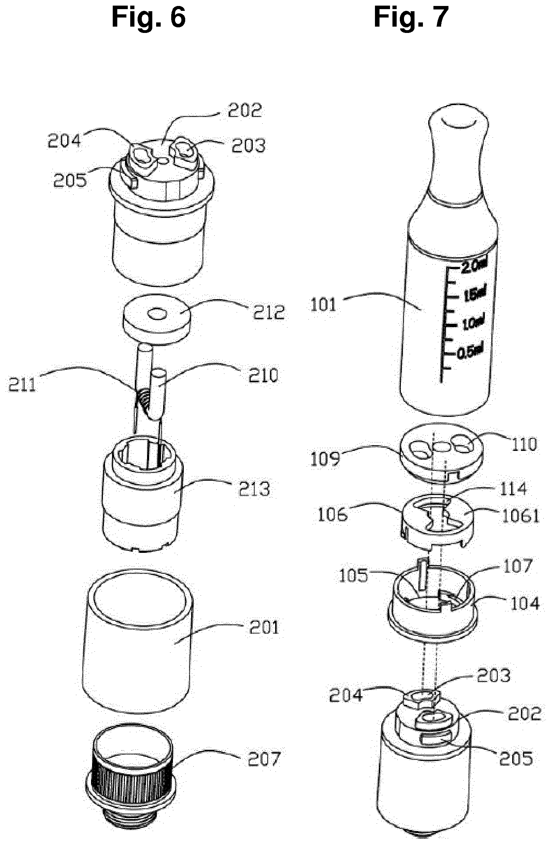

Referring to Figs. 5 and 6, which disclose the internal structure of the atomizing assembly 200. The atomizing assembly 200 comprises an outer sleeve 201, an atomizing chamber 209 is formed inside the outer sleeve 201, and the atomizing unit is provided in the atomizing chamber 109. In this embodiment, the atomizing unit comprises a liquid guide body 210 and a heating element 211 in contact with the liquid guide body 210; the liquid guide body 210 has two ends and a microporous internal structure, and e-liquid can gradually seep through the liquid guide body 210 by the capillary action arising from the microporous structure. The heating element 211 is specifically an electrical heating wire wound helically in a middle position of the liquid guide body 210. The liquid guide body 210 is fixedly installed on a seat 213, the two ends thereof being close to the liquid inlets 203 and used for absorbing e-liquid that flows in through the liquid inlets 203; the heating element 211 heats the e-liquid so that the latter evaporates to form an aerosol. The connection part 202 is located on one end of the outer sleeve 201, and the heating element 211 is aligned with the gas flow outlet 206 on the connection part 202, so the aerosol generated thereby can be smoothly discharged through the gas flow outlet 206. A threaded sleeve 207 and an electrode ring 208 are provided at the other end of the outer sleeve 201; the electrode ring 208 is insulated and is fitted inside the threaded sleeve 207, the electrode ring 208 being of a hollow structure, so air can enter the atomizing chamber 209 from the interior of the electrode ring 208. A threaded connection can be established between the atomizing assembly 200 and a power supply assembly by means of the threaded sleeve 207, and the electrode ring 208 and threaded sleeve 207, as two contact electrodes, are correspondingly connected to positive/negative electrodes on the power supply assembly, while the heating element 211 is electrically connected to the threaded sleeve 207 and the electrode ring 208.

In a preferred solution in this embodiment, a layer of a fibrous material forming a liquid storage body 212 is also provided between an end of the liquid guide body 210 and the liquid inlets 203. Both ends of the liquid guide body 210 are in contact with the liquid storage body 212, and the liquid storage body 212 can adsorb e-liquid flowing in through the liquid inlets 203 and temporarily store a certain volume of e-liquid; e-liquid adsorbed on the liquid storage body 212 can be gradually guided to the heating element 211 through the liquid guide body 210. The effect of additionally providing a layer of liquid storage body 212 below the liquid inlets 203 is to prevent an excessive amount of e-liquid from flowing into the atomizing chamber 209. In another, alternative solution, no liquid storage body 212 is provided between the ends of the liquid guide body 210 and the liquid inlets 203; instead, the two ends of the liquid guide body 210 extend directly into the liquid inlets 203, and completely fill the space of the liquid inlets 203.

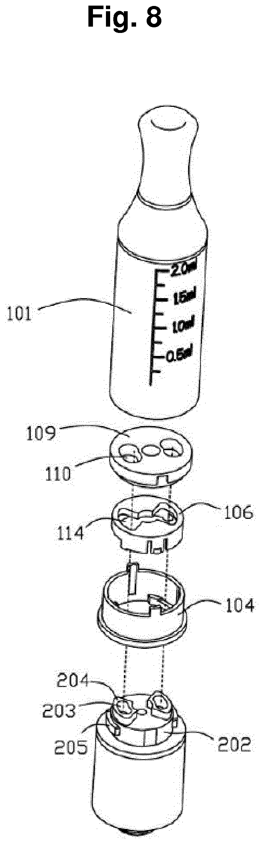

Referring to Figs. 7 and 8, these show schematic diagrams of the states of various components when the connection part is in the initial position and the engaged position respectively. During rotation, the positions of the housing 101 on the liquid storage device 100 and of the seal 109 and connection ring 104 do not change; the only change is in the position, in the circumferential direction, of the rotary component 106 following the connection part 202. It can be seen from Fig. 7 that when the protruding engagers 205 on the connection part 202 can be inserted as guided by the guide slots 105, the projecting parts 204 are positioned in alignment with the accommodating holes 114 on the rotary component 106 and insert into the accommodating holes 114; at this time, the positions of the accommodating holes 114 are staggered with respect to the positions of the liquid outlets 110, and an end face 1061 of the rotary component 106 tightly abuts the liquid outlets 110 to seal the e-liquid. It can be seen from Fig. 8 that when the connection part 202 has been rotated by 90 degrees, the protruding engagers 205 engages and connects with the steps 107, and at the same time the rotary element 106 has also rotated by 90 degrees, so that the accommodating holes 114 are aligned with the liquid outlets 110, the liquid outlets 110 are opened, and at the same time the projecting parts 204 abut the liquid outlets 110, so that the liquid outlets 110 are connected with the liquid inlets 203. When the connection part 202 rotates back to the initial position, the connection part 202 and the rotary component 106 return to the state shown in Fig. 7.

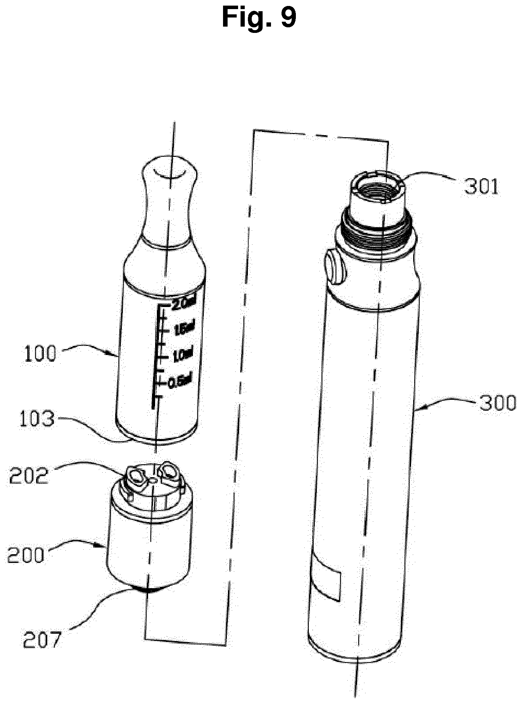

Referring to Fig. 9, this embodiment also provides an electronic cigarette, which only needs to consist of three parts, namely a liquid storage device 100, an atomizing assembly 200 and a power supply assembly 300 which are connected in sequence, to form a rod shape overall. As stated above, the atomizing assembly 200 has the connection part 202 at one end and the threaded sleeve 207 at the other end; one end of the atomizing assembly 200 engages and connects with the open end 103 of the liquid storage device 100 by means of the connection part 202. A threaded part 301 is provided on one end of the power supply assembly 300, a threaded connection is formed between the other end of the atomizing assembly 200 and the threaded part 301 by means of the threaded sleeve 207, and at the same time the power supply assembly 300 provides an operating power source for the atomizing unit inside the atomizing assembly 200.

## Claims

1. A replaceable liquid storage device (100) that is connectable to an atomizing assembly (200), the interior of the liquid storage device (100) being configured to store e-liquid, wherein the liquid storage device (100) has an open end (103) on which are provided a seal (109) having a liquid outlet (110) and a rotary component (106) which abuts the seal (109); wherein a connection ring (104) is fixedly fitted onto the open end (103), the rotary component (106) is received in the connection ring (104) so as to be capable of relative rotation, wherein on an inner wall of the connection ring (104) guide slots (105) for engaging with protruding engagers (205) of a connection part (202) of the atomizing assembly (200) are provided so that when the liquid storage device (100) is not connected to the atomizing assembly (200), the rotary component (106) blocks the liquid outlet (110) so as to seal e-liquid inside; when the liquid storage device (100) is connected to the atomizing assembly (200), the rotary component (106) can rotate and open the liquid outlet (110).

2. The liquid storage device (100) as claimed in claim 1, **characterized in that** the housing (101) of the liquid storage device (100) is made of a transparent material.

## Patentansprüche

1. Auswechselbare Flüssigkeitsspeichervorrichtung (100), die mit einer Zerstäuberanordnung (200) verbindbar ist, wobei das Innere der Flüssigkeitsspeichervorrichtung (100) dazu ausgelegt ist, ein E-Liquid zu lagern, wobei die Flüssigkeitsspeichervorrichtung (100) ein offenes Ende (103) hat, an dem eine Dichtung (109) mit einem Flüssigkeitsauslass (110) und einer drehbaren Komponente (106) vorgesehen ist, die an der Dichtung (109) anliegt; wobei ein Verbindungsring (104) fest auf dem offenen Ende (103) angebracht ist, wobei die drehbare Komponente (106) in dem Verbindungsring (104) aufgenommen ist, sodass sie zu einer relativen Drehbewegung fähig ist, wobei an einer inneren Wand des Verbindungsrings (104) Führungsschlitze (105) zum Eingriff mit vorstehenden Kontakten (205) eines Verbindungsteils (202) der Zerstäuberanordnung (200) vorgesehen sind, sodass die Flüssigkeitsspeichervorrichtung (100) nicht mit der Zerstäuberanordnung (200) verbunden ist, wobei die drehbare Komponente (106) den Flüssigkeitsauslass (110) blockiert, sodass das E-Liquid darin abgedichtet wird; wenn die Flüssigkeitsspeichervorrichtung (100) mit der Zerstäuberanordnung (200) verbunden ist, kann die drehbare Komponente (106) sich um den Flüssigkeitsauslass (110) drehen und ihn öffnen.

2. Flüssigkeitsspeichervorrichtung (100) nach Anspruch 1, **dadurch gekennzeichnet, dass** das Gehäuse (101) der Flüssigkeitsspeichervorrichtung (100) aus einem transparenten Material hergestellt ist.

## Revendications

1. Dispositif de stockage de liquide remplaçable (100) qui peut être connecté à un ensemble d'atomisation (200), l'intérieur du dispositif de stockage de liquide (100) étant configuré pour stocker du liquide pour des cigarettes électroniques, dans lequel le dispositif de stockage de liquide (100) a une extrémité ouverte (103) sur laquelle sont prévus un joint (109) ayant une sortie de liquide (110) et un composant rotatif (106) qui vient en butée contre le joint (109) ; dans lequel une bague de connexion (104) est montée de manière fixe sur l'extrémité ouverte (103), le composant rotatif (106) est reçu dans la bague de connexion (104) de manière à permettre une rotation relative, dans lequel sur une paroi intérieure de la bague de connexion (104), des fentes de guidage (105) destinées à s'engager avec des dispositifs de prise en saillie (205) d'une partie de connexion (202) de l'ensemble d'atomisation (200) sont prévues de sorte que lorsque le dispositif de stockage de liquide (100) n'est pas connecté à l'ensemble d'atomisation (200), le composant rotatif (106) bloque la sortie de liquide (110) de manière à sceller le liquide pour des cigarettes électronique à l'intérieur ; lorsque le dispositif de stockage de liquide (100) est connecté à l'ensemble d'atomisation (200), le composant rotatif (106) peut tourner et ouvrir la sortie de liquide (110).

2. Dispositif de stockage de liquide (100) selon la revendication 1, **caractérisé en ce que** le logement (101) du dispositif de stockage de liquide (100) est fabriqué en un matériau transparent.
